Europäisches Patentamt

**European Patent Office**

Office européen des brevets

(19)

(11) Numéro de publication: **0 055 651**
**B1**

(12) **FASCICULE DE BREVET EUROPEEN**

(45) Date de publication du fascicule du brevet:
**10.10.84**

(21) Numéro de dépôt: **81401979.0**

(22) Date de dépôt: **11.12.81**

(51) Int. Cl.³: **B 01 J 27/02,** B 01 J 23/26,
B 01 J 21/18, B 01 J 35/10,
C 07 C 17/20, C 07 C 19/08

(54) Catalyseurs de fluoruration en phase gazeuse des dérivés chlorés aliphatiques, à base de charbon actif imprégné de sulfate de chrome, et procédés de fluoruration utilisant ces catalyseurs.

(30) Priorité: **29.12.80 FR 8027660**

(43) Date de publication de la demande:
**07.07.82 Bulletin 82/27**

(45) Mention de la délivrance du brevet:
**10.10.84 Bulletin 84/41**

(84) Etats contractants désignés:
**BE DE FR GB IT NL**

(56) Documents cités:
**FR - A - 2 111 865**
**FR - A - 2 433 500**
**US - A - 2 005 706**
**US - A - 3 600 450**

(73) Titulaire: **ATOCHEM, 12/16, allée des Vosges,
F-92400 Courbevoie (FR)**

(72) Inventeur: **Foulletier, Louis, 5, chemin Croix-Berthet,
F-69600 Oullins (FR)**

(74) Mandataire: **Rochet, Michel et al, ATOCHEM Service
PROPRIETE INDUSTRIELLE Tour Manhattan Cédex 21,
F-92091 Paris la Défense (FR)**

BUNDESDRUCKEREI BERLIN

## 0 055 651

### Description

La présente invention concerne des catalyseurs de fluoruration en phase ganzeuse des dérivés chlorés aliphatiques saturés par l'acide fluorhydrique anhydre, constitués par un support de charbon actif imprégné de sulfate de chrome. L'invention concerne également des procédés de fluoruration des dérivés chlorés aliphatiques saturés mettant en jeu ces catalyseurs dans des réacteurs à lit fluidisé.

De très nombreux catalyseurs ont été proposés pour ces réactions des fluoruration par substitution d'atomes de fluor à des atomes de chlore. Ce sont le plus souvent des oxydes ou des halogénures de chrome, d'aluminium, de cobalt, de fer, de titane, de nickel, de cuivre, de palladium ou de zirconium, utilisés tels quels ou sur des supports divers, comme les charbons actifs et les alumines.

Le brevet français n° 720 474 et son certificat d'addition n° 43 972 enseignent la fluoruration par l'acide fluorhydrique, en phase gazeuse, d'hydrocarbures contenant un halogène autre que le fluor sur des catalyseurs à base de charbon de bois, imprégné ou non d'halogénures métalliques autres que les halogénures d'antimoine, déjà connus comme catalyseurs de fluoruration en phase liquide.

Le brevet britannique n° 2 210 369 revendique la fluoruration d'halohydrocarbures de $C_1$ à $C_3$ sur des catalyseurs à base d'halogénures de chrome déposés sur coke ou sur charbon actif.

Le brevet des Etats-Unis n° 2 458 551 décrit un catalyseur préparé par imprégnation de charbon actif avec du trichlorure de chrome et chauffage avec de l'acide fluorhydrique dans des conditions anhydres.

Pour l'obtention de trichlorotrifluoréthane et de dichlorotétrafluoréthane à faible teneur en isomères asymétriques, le brevet français n° 2 000 688 préconise l'emploi, comme catalyseur de la réaction du tétrachloréthylène avec le chlore et l'acide fluorhydrique, du trifluorure de chrome pur ou supporté sur charbon de bois, coke de pétrole ou coke de houille.

Le brevet des Etats-Unis N° 3 600 450 décrit un procédé de production du fluorure de vinylidène par réaction en phase gazeuse du chlorure de vinylidène avec l'acide fluorhydrique, en présence d'un catalyseur à base d'un sel de chrome trivalent, choisi dans le groupe constitué par le chlorure, le bromure, le fluorure, le sulfate, le nitrate, le phosphate, le formiate et l'acétate de chrome. Ce catalyseur, qui peut être supporté ou non par un charbon actif, a l'avantage d'inhiber la fixation d'acide fluorhydrique sur la double liaison du chlorure de vinylidène.

Tous ces catalyseurs aux sels de chrome déposés sur des supports carbonés conviennent plus ou moins bien à la fluoruration en phase gazeuse des hydrocarbures chlorés ou chlorofluorés aliphatiques dans des réacteurs à lit fixe. Ils sont par contre très mal adaptés à la fluoruration dans des réacteurs à lit fluidisé, qui exigent des particules de forme régulière et de granulométrie homogène. Le simple broyage des catalyseurs, suivi d'un tamisage pour sélectionner les particules de taille convenable, donne des particules de forme irrégulière et conduit à des déchets importants de catalyseur.

La demanderesse a découvert que les catalyseurs de fluoruration en phase gazeuse s'empoisonnent principalement par formation de goudrons à leur surface. L'emploi de réacteurs à lit fluidisé, provoquant l'abrasion des grains de catalyseur, permet d'éliminer ces goudrons et de maintenir l'activité du catalyseur. Celui-ci se consomme uniquement par attrition, et il n'est plus besoin d'arrêter l'installation pour recharger le réacteur en catalyseur.

Les catalyseurs aux sels de chrome déposés sur charbon actif de l'art antérieur présentent également un ou plusieurs des inconvénients suivants:

— faible taux de transformation de l'acide fluorhydrique
— faible productivité
— faible sélectivité
— faible activité dans la fluoruration de dérivés chlorés autres que les chlorocarbures, et en particulier dans la fluoruration des nitriles chlorés.

Selon l'invention on obvie à tous ces inconvénients en préparant des catalyseurs de fluoruration en phase gazeuse par imprégnation au moyen d'une solution de sulfate de chrome d'un charbon actif possédant une surface spécifique totale supérieure à 1000 $m^2/g$ et une mésoporosité et une macroporosité élevées. La mésoporosité, définie par la surface des pores de 4 à 5 nm de rayon, doit être supérieure à 5 $m^2/g$. La macroporosité, définie par la surface des pores dont le rayon est égal ou supérieur à 25 nm, doit être supérieure à 2 $m^2/g$.

Le choix de l'anion du sel de chrome servant à l'imprégnation du charbon actif est particulièrement critique pour la spécificité du catalyseur. La demanderesse a découvert, de façon tout-à-fait inattendue, que les ions sulfate conduisent à des catalyseurs capables de permettre la fluoruration avec de bons rendements des nitriles chlorés aliphatiques, comme le trichloroacétonitrile. En l'absence de ces ions sulfate, les taux de transformation sont beaucoup plus faibles.

Au lieu d'utiliser une solution de sulfate de chrome pour l'imprégnation du charbon actif, on peut employer une solution aqueuse de trioxyde de chrome $CrO_3$ contenant de l'acide sulfurique. Une partie au moins du chrome hexavalent est réduite en chrome trivalent par le charbon actif.

Les catalyseurs selon l'invention ont une teneur en chrome comprise entre 0,5 et 1,5 atome-gramme

2

par litre de catalyseur. Ils sont constitués de particules dont le diamètre est compris entre 100 µm et 3000 µm.

Les exemples suivants, donnés à titre non limitatif, illustrent divers modes de préparation des catalyseurs selon l'invention et l'utilisation de ces catalyseurs dans diverses réactions de fluoruration.

## Exemple 1

On utilise comme support un charbon de bois présentant les caractéristiques suivants:

| | |
|---|---|
| Densité | 0,55 |
| Surfaces spécifique totale | 1200 m$^2$/g |
| Surface des pores $\geq$ 25 nm | 3 m$^2$/g |
| Surface des pores 4—5 nm | 9 m$^2$/g |

On imprègne ce charbon actif avec une solution aqueuse de sulfate de chrome pur, à la concentration de 600 g/l, de manière à avoir un atome-g de chrome par litre de catalyseur. Le catalyseur est séché à 150° C en lit fluidisé.

Ce catalyseur est utilisé pour la fluoruration en phase gazeuse du trichlorotrifluoréthane en dichlorotétrafluoréthane, dans un réacteur à lit fluidisé fonctionnant dans les conditions suivantes:

| | |
|---|---|
| Rapport molaire HF/C$_2$Cl$_3$F$_3$ | 1,02/1 |
| Débit | 18 moles/h/l |
| Température | 400° C |

Le taux de transformation de l'acide fluorhydrique est de 75%. Le dichlorotétrafluoréthane obtenu contient 85% d'isomère symétrique CClF$_2$—CClF$_2$.

## Exemple 2

On prépare un catalyseur analogue à celui de l'exemple 1, mais avec une teneur de 0,75 atome-g de chrome par litre de catalyseur.

Ce catalyseur est utilisé, dans un réacteur à lit fluidisé, pour la fluoruration du trichlorotrifluoréthane en dichlorotétrafluoréthane, dans les conditions suivantes:

| | |
|---|---|
| Rapport molaire HF/C$_2$Cl$_3$F$_3$ | 1,45/1 |
| Débit | 11 moles/h/l |
| Température | 400° C |

Le taux de transformation de l'acide fluorhydrique est de 67% et le dichlorotétrafluoréthane obtenu contient 85% d'isomère symétrique.

## Exemple 3

Le catalyseur de l'exemple 1 est utilisé dans la réaction de fluoruration de l'hexachloréthane, formé in situ par réaction du chlore sur le tétrachloréthylène.

Sur le catalyseur, maintenu à 400° C, on fait passer un mélange d'acide fluorhydrique, de chlore et de tétrachloréthylène dans le rapport molaire 3,97/1,2/1, avec un débit de 18,39 moles/h/l.

Le taux de transformation de l'acide fluorhydrique est de 85,4%. Les taux de transformation du tétrachloréthylène sont de:

— 44,6% en trichlorotrifluoréthane, contenant 98,5% d'isomère symétrique CClF$_2$—CCl$_2$F
— 38,3% en dichlorotétrafluoréthane, contenant 82,7% d'isomère symétrique.

## Exemple 4 (Exemple comparatif)

Suivant les données du brevet français n° 2 000 688, on imprègne 100 g d'un charbon actif, dérivé du pétrole et commercialisé sous la marque COLUMBIA CXX, par une solution de 61 g de trichlorure de chrome hydraté CrCl$_3$, 6 H$_2$O dans 200 g d'eau. Le catalyseur est séché à 110° C.

Sur ce catalyseur maintenu à 400° C on fait passer un mélange d'acide fluorhydrique, de chlore et de tétrachloréthylène dans le rapport molaire 4,3/1,2/1,1, avec un débit de 13 moles/h/l.

**0 055 651**

Le taux de transformation de l'acide fluorhydrique n'est que de 32,5%. Les taux de transformation du tétrachloréthylène sont de:

— 44,5% en trichlorotrifluoréthane, contenant
   57% d'isomère symétrique
— 1,4% en dichlorotétrafluoréthane, contenant
   43% d'isomère symétrique.

Exemple 5

Le catalyseur de l'exemple 1 est utilisé pour la fluoruration en lit fluidisé du trichloroacétonitrile, dans les conditions suivantes:

Rapport HF/CCl₃ CN                    5,36/1
Débit                                 4,55 moles/h/l
Température                            400°C

Le taux de transformation de l'acide fluorhydrique est de 49,9%. Les taux de transformation du trichloroacétonitrile sont de:

— 45,2% en trifluoracétonitrile
— 28,4% en chlorodifluoracétonitrile.

Exemple 6

On prépare un catalyseur en utilisant 1 litre du charbon actif de l'exemple 1 et en l'imprégnant par 400 cm³ d'une solution aqueuse contenant 1,0 mole de trioxyde de chrome CrO₃ et 1,83 mole d'acide sulfurique. La totalité de la solution est adsorbée par le charbon actif. Le catalyseur est séché à 150°C en lit fluidisé.

Ce catalyseur est utilisé pour la fluoruration en lit fluidisé du trichloroacétonitrile, dans les conditions suivantes:

Rapport HF/CCl₃ CN                    6,95/1
Débit                                 5,71 moles/h/l
Température                            400°C

On obtient un taux de transformation de 38,7% pour l'acide fluorhydrique. Les taux de transformation du trichloroacétonitrile sont de:

— 54,1% en trifluoroacétonitrile
— 24,9% en chlorodifluoroacétonitrile.

Exemple 7 (Exemple comparatif)

Cet exemple montre le rôle déterminant des ions $SO_4^-$ pour l'aptitude du catalyseur à favoriser la fluoruration du trichloroacétonitrile.

On prépare un catalyseur analogue à celui de l'exemple 6 en imprégnant 1 litre du charbon actif utilisé à l'exemple 1 avec 400 cm³ d'une solution aqueuse de 2,5 moles de trioxyde de chrome CrO₃, ne contenant pas d'acide sulfurique. La totalité de la solution est adsorbée par le charbon. Le catalyseur est séché à 150°C en lit fluidisé.

Ce catalyseur est utilisé pour la fluoruration du trichloroacétonitrile en lit fluidisé, dans les conditions suivantes:

Rapport HF/CCl₃ CN                    4,95/1
Débit                                 4,2 moles/h/l
Température                            400°C

Le taux de transformation de l'acide fluorhydrique n'est que de 21,8%. Les taux de transformation du trichloroacétonitrile sont de:

— 2,8% en trifluoroacétonitrile
— 7,7% en chlorodifluoroacétonitrile.

4

**0 055 651**

1. Catalyseurs de fluoruration en phase gazeuse des dérivés chlorés aliphatiques saturés, à base de sulfate de chrome déposé sur un support de charbon actif, caractérisés en ce que le support de charbon actif présente une surface spécifique totale supérieure à 1000 m²/g, une surface des pores de 4 à 5 nm de rayon supérieure à 5 m²/g et une surface des pores de rayon égal ou supérieur à 25 nm supérieure à 2 m²/g.

2. Catalyseurs selon la revendication 1, caractérisé en ce que la teneur en chrome est comprise entre 0,5 et 1,5 atome-g par litre de catalyseur.

3. Catalyseurs selon l'une des revendications 1 ou 2, caractérisés en ce qu'ils sont constitués de particules de diamètres compris entre 100 μm et 3000 μm.

4. Procédé de préparation des catalyseurs selon l'une quelconque des revendications 1 à 3, caractérisé en ce que le support de charbon actif est imprégné par une solution aqueuse de trioxyde de chrome contenant de l'acide sulfurique, puis séché à une température d'environ 150°C.

5. Procédé de fluoruration en phase gazeuse des dérivés chlorés aliphatiques saturés, caractérisé par l'utilisation dans un réacteur à lit fluidisé d'un catalyseur selon l'une ou l'autre des revendications 1 à 3.

## Patentansprüche

1. Katalysator zur Gasphasenfluorierung von gesättigten aliphatischen Chlorverbindungen auf der Basis von Chromsulfat auf einem Aktivkohleträger, dadurch gekennzeichnet, daß der Aktivkohleträger eine gesamte spezifische Oberfläche über 1000 m²/g, eine Oberfläche der Poren mit einem Radius von 4 bis 5 nm über 5 m²/g und eine Oberfläche der Poren eines Radius gleich oder größer 25 nm über 2 m²/g hat.

2. Katalysatoren nach Anspruch 1, dadurch gekennzeichnet, daß der Chromgehalt 0,5 bis 1,5 Grammatom pro Liter Katalysator beträgt.

3. Katalysatoren nach Anspruch 1 oder 2, dadurch gekennzeichnet, daß sie aus Teilchen eines Durchmessers von 100 μm bis 3000 μm bestehen.

4. Verfahren zur Herstellung der Katalysatoren nach irgendeinem der Ansprüche 1 bis 3, dadurch gekennzeichnet, daß der Aktivkohleträger mit einer wäßrigen Lösung von Chromtrioxid getränkt wird, die Schwefelsäure enthält, und dann bei einer Temperatur von etwa 150°C getrocknet wird.

5. Verfahren zur Gasphasenfluorierung von gesättigten aliphatischen Chlorverbindungen, gekennzeichnet durch die Verwendung eines Katalysators nach irgendeinem der Ansprüche 1 bis 3 in einem Wirbelbettreaktor.

## Claims

1. Catalysts for the gas phase fluorination of saturated aliphatic chlorinated derivatives, the catalyst being based on chromium sulphate deposited on an active charcoal carrier, characterised in that the active charcoal carrier has a total specific surface area greater than 1,000 m²/g, a surface of the pores of 4—5 nm radius greater than 5 m²/g and a surface of the pores of radius equal to or greater than 25 nm greater than 2 m²/g.

2. Catalysts according to Claim 1, characterised in that the chromium content is between 0.5 and 1.5 g-atom per litre of catalyst.

3. Catalysts according to one of Claims 1 or 2, characterised in that they consists of particles of diameters between 100 μm and 3,000 μm.

4. Process for the preparation of the catalysts according to any one of Claims 1 to 3, characterised in that the active charcoal carrier is impregnated with an aqueous chromium trioxide solution containing sulphuric acid and is then dried at a temperature of about 150°C.

5. Process for the gas phase fluorination of saturated aliphatic chlorinated derivatives, characterised in that a catalyst according to one or other of Claims 1 to 3 is used in a fluidised bed reactor.